Europäisches Patentamt

European Patent Office

Office européen des brevets

(11) Publication number: **0 357 296**
**A1**

## EUROPEAN PATENT APPLICATION

(21) Application number: 89308330.3

(22) Date of filing: 16.08.89

(51) Int. Cl.5: **A61K 37/54** , //(**A61K37/54, 37:465**)

The title of the invention has been amended (Guidelines for Examination in the EPO, A-III, 7.3).

(30) Priority: 17.08.88 GB 8819607

(43) Date of publication of application:
07.03.90 Bulletin 90/10

(84) Designated Contracting States:
BE CH DE FR GB IT LI NL SE

(71) Applicant: THE WELLCOME FOUNDATION LIMITED
183-193 Euston Road
London NW1 2BP(GB)

(72) Inventor: Berger, Henry, Jr.
610 Normandy Street
Cary North Carolina 27511(US)

(74) Representative: Stott, Michael John et al
The Wellcome Research Laboratories Group
Patents & Agreements Langley Court
Beckenham Kent BR3 3BS(GB)

(54) Combination of t-PA and protein C.

(57) The use of t-PA and activated protein C in preventing reocclusion of a blood vessel in a mammal.

EP 0 357 296 A1

## NOVEL COMBINATION

The present invention relates to a combination of tissue plasminogen activator and activated protein C, to pharmaceutical formulations containing them, and to their use in human and veterinary medicine.

There exists a dynamic equilibrium between the enzyme system capable of forming blood clots, the coagulation system, and the enzyme system capable of dissolving blood clots, the fibrinolytic system, which maintains an intact patent vascular bed. To limit loss of blood from injury, blood clots are formed in the injured vessel. After natural repair of the injury, the superfluous blood clots are dissolved through operation of the fibrinolytic system. Occasionally, blood clots form without traumatic injury and may lodge in major blood vessels resulting in a partial or even total obstruction to blood flow. When this occurs in the heart, lung or brain, the result may be a myocardial infarction, pulmonary embolism or stroke. These conditions combined are the leading cause of morbidity and mortality in the industrialised nations.

Blood clots consist of a fibrous network that is capable of dissolution by the proteolytic enzyme plasmin. The enzyme is derived from the inactive proenzyme, plasminogen, a component of blood plasma, by the action of a plasminogen activator. There are two immunologically distinct mammalian plasminogen activators. Intrinsic plasminogen activator, also known as urokinase, is an enzyme produced by the kidney and can be isolated from urine. It can also be prepared from a number of tissue culture sources. Extrinsic plasminogen activator, also known as vascular plasminogen activator and as tissue plasminogen activator (t-PA), can be isolated from many tissue homogenates (notably human uterus), the vascular cell wall and from some cell cultures. In addition to these two kinds of plasminogen activator, there is also a bacterial product, streptokinase, prepared from beta-haemolytic streptococci. A major drawback with both urokinase and streptokinase is that they are active throughout the circulation and not just at the site of a blood clot. In contrast, the biological activity of t-PA is dependent on the presence of fibrin to which it binds and where it is activated. Maximum activity is thus developed only at the site of a blood clot, i.e. in the presence of the fibrin network to be dissolved, and this greatly reduces the risk of haemorrhage.

Protein C is a vitamin K-dependent zymogen that occurs naturally in the blood plasma. It is inactive in its circulating form but is converted to an active form, otherwise known as activated protein C, by the proteolytic cleavage of a dodecapeptide from the N-terminus. In vivo proteolysis is brought about at the endothelial surface by a thrombin/thrombomodulin cofactor pathway. Proteolysis of the inactive form of protein C can also be obtained in vitro by various proteases. Activated protein C has two physiological actions. It is profibrinolytic in that it inactivates plasminogen activator inhibitors and in that it may induce the release of plasminogen activator in vivo (1-6). It is also an anticoagulant in that it engenders proteolytic inactivation of the activated clotting factors Va and VIIIa (6-9).

One of the main problems perceived with thrombolytic therapy with t-PA, as indeed with other plasminogen activators, is the extent to which reocclusion of a blood vessel may occur following thrombolysis. Various drugs including heparin (10 & 11), antiplatelet agents (12), nitrates (15) and t-PA itself (13 & 14) have been used to maintain patency of the blood vessel. However, a more effective treatment in this regard is still sought.

It has now been found that a combination of t-PA and activated protein C has a synergistic effect in preventing reocclusion of a blood vessel. Accordingly the present invention provides combination of t-PA and activated protein C for use in preventing reocclusion of a blood vessel in a mammal.

The t-PA of use with the present invention may be any bioactive protein substantially corresponding to mammalian, and especially human, t-PA and includes forms with and without glycosylation. It may be one- or two-chain t-PA, or a mixture thereof, as described in EP-A-112 122, and, in the case of fully glycosylated human t-PA, has an apparent molecular weight as determined by polyacrylamide gel electrophoresis of about 70,000 and an isoelectric point of between 7.5 and 8.0. Preferably, the t-PA has a specific activity of about 300,000 to 600,000 IU/mg. As used herein, an International Unit (IU) is the International Unit of activity as defined by WHO, National Institute for Biological Standards and Control, Holly Hill, Hampstead, London, NW₃ 6RB, UK and determined by comparison with a standard preparation using a clot lysis assay.

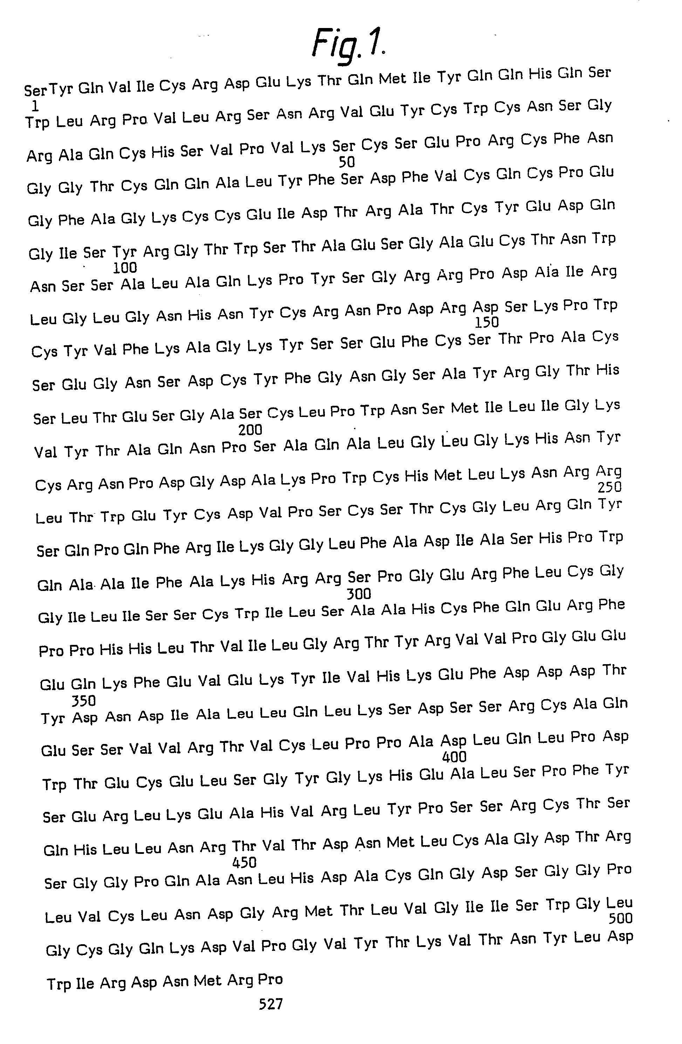

The amino acid sequence of t-PA preferably substantially corresponds to that set forth in Figure 1. The sequence may thus be identical to that in Figure 1 or contain one or more amino acid deletions, substitutions, insertions, inversions or additions of allelic origin or otherwise, the resulting sequence having at least 80%, and preferably 90%, homology with the sequence in Figure 1 and retaining essentially the same biological and immunological properties of the protein. In particular, the sequence may be identical to that in Figure 1 or may be the same but with the amino acid in the 245th position from the serine N-terminus being valine instead of methionine, either sequence optionally being without any of the first three amino acids or optionally having an additional polypeptide N-terminal presequence of Gly-Ala-Arg.

2

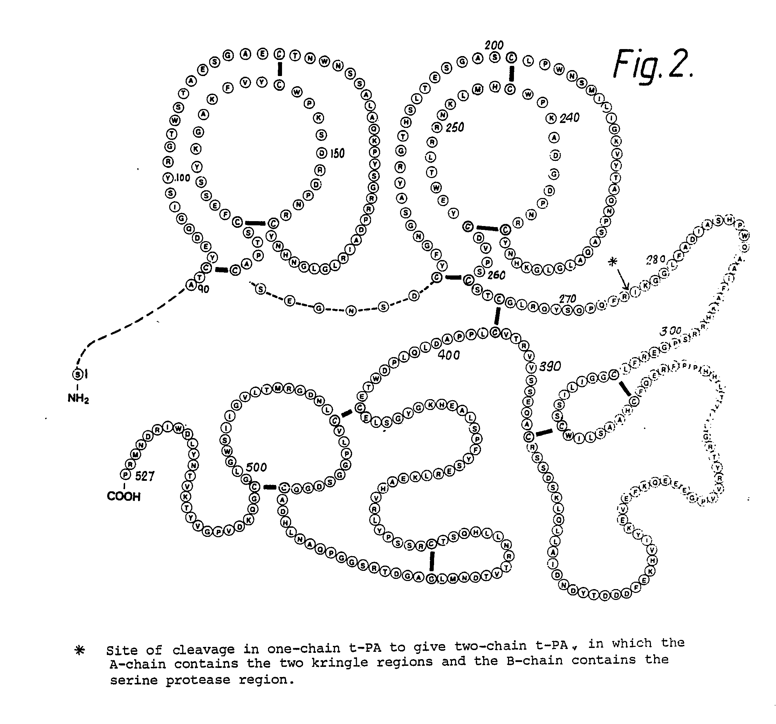

The amino acid sequence set forth in Figure 1 has thirty five cysteine residues and thus the potential for forming seventeen disulphide bridges. Based on analogy with other proteins whose structure has been determined in more detail, the postulated structure for the sequence (arising from disulphide bond formation) between the amino acid in the 90th position and the proline C-terminus is set forth in Figure 2. The structure of the N-terminal region is less certain although some proposals have been put forward (Progress in Fibrinolysis, 1983, 6, 269-273; and Proc. Natl. Acad. Sci., 1984, 81 5355-5359). The most important features of the structure of t-PA are the two kringle regions (between the 92nd and the 173rd amino acids and between the 180th and 261st amino acids), which are responsible for the binding of the protein to fibrin, and the serine protease region, which comprises the major part of the B-chain and which is responsible for the activation of plasminogen. The amino acids of special significance in serine proteases are the catalytic triad, His/Asp/Ser. In t-PA these occur at the 322nd, the 371st and the 463rd positions. The disulphide bridge between the 264th and 395th cysteine amino acid residues is also important in that it holds together the A- and the B-chains in the two-chain form of t-PA.

In Figures 1 and 2, the conventional one and three letter codes have been employed for the amino acid residues as follows:-

Asp D Aspartic acid
Thr T Threonine
Ser S Serine
Glu E Glutamic acid
Pro P Proline
Gly G Glycine
Ala A Alanine
Cys C Cysteine
Val V Valine
Ile I Isoleucine
Leu L Leucine
Tyr Y Tyrosine
Phe F Phenylalanine
His H Histidine
Arg R Arginine
Lys K Lysine
Trp W Tryptophan
Gln Q Glutamine
Met M Methionine
Asn N Asparagine

The t-PA may be obtained by any of the procedures described or known in the art. For example, it may be obtained from a normal or neoplastic cell line of the kind described in Biochimica et Biophysica Acta, 1979, 580, 140-153; EP-A-41 766; EP-A-113 319 or EP-A-227 102. It is preferred, however, that t-PA is obtained from a cultured transformed or transfected cell line derived using recombinant DNA technology as described in, for example, EP-A-93 619; EP-A-117 059; EP-A-117 060; EP-A-173 552; EP-A-174 835; EP-A-178 105; EP-A-225 177; EP-A-225 286; EP-A-227 064; EP-A-237 157; EP-A-245 949; WO 86/01538; WO 86/05514; or WO 86/05807. It is particularly preferred that Chinese hamster ovary (CHO) cells are used for the production of t-PA and are derived in the manner as described in Molecular and Cellular Biology, 1985, 5(7), 1750-1759. In this way, the cloned gene is cotransfected with the gene encoding dihydrofolate reductase (dhfr) into dhfr⁻ CHO cells. Transformants expressing dhfr are selected on media lacking nucleosides and are exposed to increasing concentrations of methotrexate. The dhfr and t-PA genes are thus coamplified leading to a stable cell line capable of expressing high levels of t-PA.

The t-PA is, preferably, purified using any of the procedures described or known in the art, such as the procedures described in Biochimica et Bionhysica Acta, 1979, 580, 140-153; J.Biol.Chem., 1979, 254(6), 1998-2003; ibid, 1981, 256(13), 7035-7041; Eur.J.Biochem, 1983, 132, 681-686; EP-A-23860; EP-A-41 766; EP-A-113 319; EP-A-167 152; WO/00615; or GB-A-2 122 219.

The activated protein C of use with the present invention may be any bioactive protein substantially corresponding to mammalian, and especially human, activated protein C and includes forms with and without glycosylation. It preferably contains nine γ-carboxyglutamic acid residues and one γ-hydroxyaspartic acid residue. It may exist in a one- or two-chain form. In the case of fully glycosylated protein C, it has an apparent molecular weight as determined by polyacrylamide gel electrophoresis of about 62,000, the molecular weight of the heavy chain of the two-chain form being about 41,000 and that of the light chain being about 21,000, and an isoelectric point of 4.4-4.8.

The amino acid sequence of activated protein C preferably substantially corresponds to that described by Foster et al, (Proc.Natl.Acad.Sci, 1985, 82, 4673-4677) or by Beckman et al (Nuc.Acids.Res, 1985, 13, 5233-5247). The sequence is thus identical to that in either reference or contains one or more amino acid deletions, substitutions, insertions, inversions or additions of allelic origin or otherwise, the resulting sequence having at least 80%, and preferably 90%, homology with the sequence of either reference and retaining essentially the same biological and immunological properties of the protein.

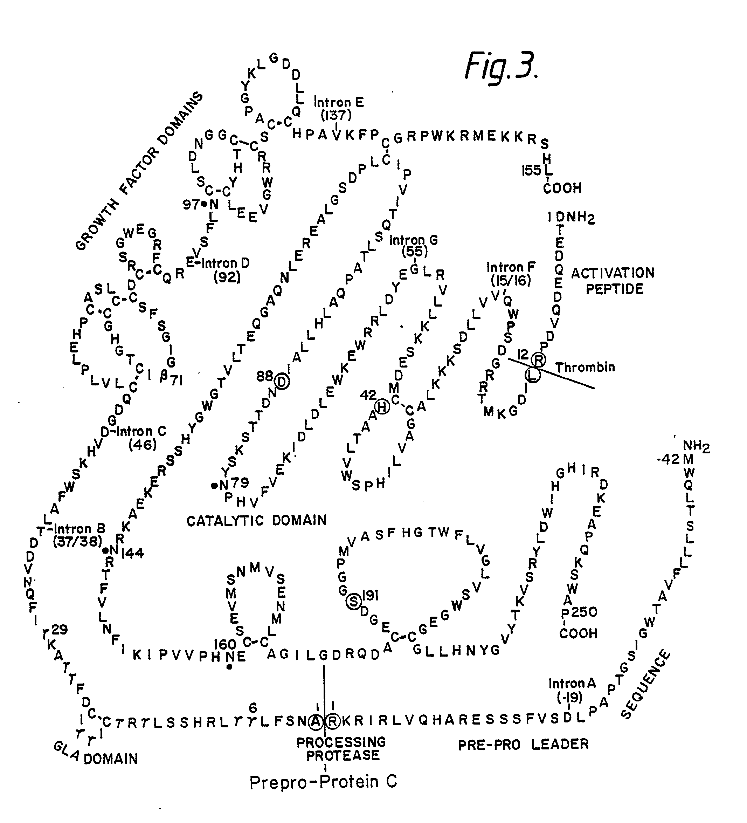

Activated protein C contains twenty-four cysteine residues with the potential to form twelve disulphide bridges. By analogy with the structure of other Vitamin K-dependent proteins, the postulated structure has been determined (Proc.Natl.Acad.Sci. 1985, 82, 4763-77) and is set out in Figure 3. It contains a GLA domain, epidermal growth factor domains and a catalytic domain, the latter containing the active site composed of Asp 88, His 42 and Ser 191. The light and heavy chains are joined between Cys 141 of the light chain and Cys 108 of the heavy chain.

Activated protein C may be obtained by any of the procedures described or known in the art. For example, it may be isolated from human plasma by conventional procedures (Kisiel et al, Methods in Enzymology, 1981, 80, 320-332; or Stenflo, J.Biol.Chem., 1976, 251, 355-363). Alternatively, it may be obtained using recombinant DNA technology by expression in a suitable host of the cloned DNA (EP-A-191 606 and EP-A-215 548). In either case, protein C is used in its activated form and this may be obtained by proteolysis using thrombin, trypsin or a protease from Russell's viper venom (Orthner et al, Biochemistry, 1988, 27, 2558-2564. Activated protein C is, preferably, purified using procedures described or known in the art.

In using t-PA and activated protein C in the manner of the present invention, it is preferred to employ them in the form of a medicament or pharmaceutical formulation. The active ingredients may be provided for use with the present invention as separate medicaments or formulations or as a single combined medicament or formulation although in the latter case both active ingredients must of course be stable and mutually compatible. The present invention also provides a pharmaceutical formulation, which comprises t-PA and activated protein C and a pharmaceutically acceptable carrier.

Generally, t-PA and activated protein C will be administered by the intravascular route, whether by infusion or by bolus injection, and thus a parenteral formulation is required. It is preferred to present a lyophilized formulation to the physician or veterinarian because of the significant transportation and storage advantages that it affords. The physician or veterinarian may then reconstitute the lyophilized formulation in an appropriate amount of solvent as and when required.

Parenteral and lyophilized pharmaceutical formulations containing t-PA are known in the art. Examples of such art include EP-A-41 766; EP-A-93 619; EP-A-112 122; EP-A-113 319; EP-A-123 304; EP-A-143 081; EP-A-156 169; EP-A-211 592; EP-A-217 379; EP-A-218 112; WO86/01104; Japanese patent publication 57-120523 (application 56-6936) and Japanese patent publication 58-65218 (application 56-163145). Additional examples include GB-A-2 176 702; GB-A-2 176 703; and GB-A-2 184 354.

Parenteral and lyophilised formulations containing activated protein C are also known in the art. Examples of such art include in particular the aforementioned EP-A-191 606.

Parenteral and lyophilized formulations containing t-PA and activated protein C together in a single, combined formulation may be prepared in a similar manner to the preparation of formulations suitable for t-PA or activated protein C per se. As mentioned before, however, the stability and mutual compatibility of the active ingredients in a particular formulation will need to be taken into account and the formulation adapted accordingly.

Intravascular infusions are normally carried out with the parenteral solution contained within an infusion bag or bottle or within an electrically operated infusion syringe. The solution may be delivered from the infusion bag or bottle to the patient by gravity feed or by use of an infusion pump. The use of gravity feed infusion systems does not afford sufficient control over the rate of administration of the parenteral solution and, therefore, the use of an infusion pump is preferred especially with solutions containing relatively high concentrations of active ingredients. More preferred, however, is the use of an electrically operated infusion system which offers even greater control over the rate of administration.

The present invention also provides a method for preventing reocclusion of a blood vessel in a mammal, which comprises administering to the mammal an effective amount of t-PA and activated protein C. In the alternative, the present invention provides a combination of t-PA and activated protein C for use in human and veterinary medicine especially for use in preventing reocclusion of a blood vessel in a mammal.

In using t-PA and activated protein C in the manner of the present invention, the active ingredients may be administered concurrently or sequentially as separate formulations or as a single combined formulation. If there is sequential administration, the delay in administering the second of the active ingredients should not be such as to lose the benefit of a potentiated anti-thrombotic effect of the combination of the active ingredients.

An effective amount of t-PA and activated protein C to remove or inhibit the formulation of a blood clot in a mammal will of course depend upon a number of factors including, for example, the age and weight of the mammal, the precise condition requiring treatment and its severity, the route of administration, the particular form of t-PA and activated protein C employed and their potency, and will ultimately be at the discretion of the attendant physician or veterinarian. It is likely, however, that an effective amount, in the case of t-PA, will be from 20,000 to 200,000 IU per kg per hour, and, in the case of activated protein C, will be from 0.01 to 0.5mg per kg per hour. Thus, for a 70 kg adult human being, an effective amount per hour will generally be, in the case of t-PA, from 1,400,000 to 14,000,000 IU, and, in the case of activated protein C, from 0.7 to 35 mg.

The following examples are provided in illustration of the present invention and should not be construed in any way as constituting a limitation thereof.

Example 1

Test Procedure

Male guinea pigs (Dunkin-Hartley) weighing 350-450 grams were anaesthetised with pentobarbital. Both jugular veins were cannulated for separate infusions of recombinant t-PA (rt-PA) and activated protein C. A 1 cm length of the right carotid artery was isolated and cleaned of connective tissue. The exposed artery was wrapped with a 1 cm$^2$ patch of absorbent paper on which was added 0.05ml of 3.7% buffered formalin solution. Immediately prior to placement of this patch, the infusion of rt-PA or its vehicle was started in one jugular vein while a bolus of 6% of the dose of activated protein C followed by continuous infusion of the remainder or an equivalent volume of its vehicle was given via the contralateral jugular vein. The incisions were closed, and the infusions were continued for a period of 5 hours. At the end of the 5 hour period the right carotid artery was re-exposed and was visually examined for the presence of a clot in the area beneath the formalin patch as evidenced by the absence of blood flow in the artery. This segment of the artery was then excised, the presence of a thrombus was confirmed by an ability to extrude a clot from the vessel. The mechanism of clot formation was previously shown to involve disruption of the endothelium resulting in platelet adhesion and aggregation followed by fibrin deposition and red cell entrapment (Fed.Proc., 1980 39, 423).

Recombinant t-PA (rt-PA) was obtained and formulated as described in GB-A-2176702. It was reconstituted and diluted in saline for infusion. Activated protein C was isolated from human plasma by barium citrate precipitation and immunoaffinity chromatography (American Red Cross). The protein was obtained in its activated form using a protease isolated from Russell's viper venom. It was provided in solution in 0.05M Tris-HCl at pH 7.4; 0.15M NaCl; 0.002M CaCl$_2$ and diluted for infusion in the same buffer containing in addition bovine serum albumin.

The proteins were infused through separate intravenous lines.

Results

The results of the separate infusions of rt-PA alone, activated protein C alone, and combined administration of rt-PA and activated protein C are summarized in Table 1 and are expressed as the ratio of the number of animals protected from thrombus formation to the total number of animals tested. Activated protein C alone at the dose indicated was not effective in protecting any animals. At the indicated dose of rt-PA alone one animal out of seven was protected. The combined administration of both agents resulted in protection in 6 of the 8 animals tested which was statistically significantly different using the Chi-squared test from the results with either agent alone (P <0.02).

5

## TABLE 1

| Group | rt-PA Dose | APC* Dose | # Protected / # Tested |
|-------|-----------|-----------|------------------------|
|       | $(10^6$ IU/kg) | (mg/kg) | |
| A     | 2         | 0         | 1/7 |
| B     | 0         | 0.43      | 0/6 |
| C     | 2         | 0.43      | 6/8 |

\* activated protein C

Conclusion

Using a combination of rt-PA and activated protein C, which when given singly offer marginal or no protection respectively against thrombosis, significant protection was observed. Thus these two proteins act synergistically as antithrombotic agents.

REFERENCES

1. Proc. Natl. Acad. Sci., 1985, 82, 1121-1125.
2. Blood, 1985, 65, 444-451.
3. Blood, 1986, 68, 1218-1223.
4. Throm. Haemostas., 1987, 57, 176-182.
5. J. Clin. Invest., 1981, 68, 1221-1228.
6. Biochemistry, 1977, 16, 5824-5831.
7. Blood, 1982, 59, 1067-1072.
8. Biochemistry, 1980, 19, 401-410.
9. Eur. J. Biochem., 1980, 107, 331-335.

10. N. Engl. J. Med., 1984, 311, 770-776.
11. Am. J. Cardiol., 1987, 59, 241-244.
12. Circulation, 1986, 73, 206-223.
13. Circulation, 1986, 73, 347-352.
14. Circulation, 1987, 76, (Suppl. IV), IV-452 (abstr. 1801).
15. N. Engl. J. Med., 1987, 317, 1055-1059.

**Claims**

1. A combination of t-PA and activated protein C for use in preventing reocclusion of a blood vessel in a mammal.

2. A combination according to claim 1, wherein the amino acid sequence of t-PA corresponds to that set forth in Figure 1 or has the same sequence but with the amino acid in the 245th position from the serine N-terminus being valine instead of methionine.

3. A combination according to either of the preceding claims, wherein the amino acid sequence of activated protein C corresponds to that set forth in Figure 3.

4. A combination according to any one of the preceding claims, wherein t-PA or activated protein C is produced using recombinant DNA technology.

5. A combination of from 20,000 to 200,000 IU per kg per hour of t-PA and from 0.01 to 0.5 mg per kg per hour of activated protein C for use in preventing reocclusion of a blood vessel in a mammal.

6. A pharmaceutical formulation comprising a combination according to any one of the preceding claims and a pharmaceutically acceptable carrier.

7. Use of t-PA and activated protein C in the manufacture of a medicament for preventing reocclusion of a blood vessel in a mammal.

8. Use according to claim 7, wherein the amino acid sequence of t-PA corresponds to that set forth in Figure 1 or has the same sequence but with the amino acid in the 245th position from the serine N-terminus being valine instead of methionine.

9. Use according to either of claims 7 and 8, wherein the amino acid sequence of activated protein C corresponds to that set forth in Figure 3.

10. Use according to any one of claims 7 to 9, wherein t-PA or activated protein C is obtained using recombinant DNA technology.

11. Use of t-PA and activated protein C in the manufacture of a medicament for preventing reocclusion of a blood vessel in a mammal by administering to the mammal from 20,000 to 200,000 IU per kg per hour of t-PA and from 0.01 to 0.5 mg per kg per hour of activated protein C.

12. Use of a combination of from 20,000 to 200,000 IU per kg per hour of t-PA and from 0.01 to 0.5 mg per kg per hour of activated protein C in preventing reocclusion of a blood vessel in a mammal.

# *Fig.1.*

```
SerTyr Gln Val Ile Cys Arg Asp Glu Lys Thr Gln Met Ile Tyr Gln Gln His Gln Ser
1
Trp Leu Arg Pro Val Leu Arg Ser Asn Arg Val Glu Tyr Cys Trp Cys Asn Ser Gly
Arg Ala Gln Cys His Ser Val Pro Val Lys Ser Cys Ser Glu Pro Arg Cys Phe Asn
                                       50
Gly Gly Thr Cys Gln Gln Ala Leu Tyr Phe Ser Asp Phe Val Cys Gln Cys Pro Glu
Gly Phe Ala Gly Lys Cys Cys Glu Ile Asp Thr Arg Ala Thr Cys Tyr Glu Asp Gln
Gly Ile Ser Tyr Arg Gly Thr Trp Ser Thr Ala Glu Ser Gly Ala Glu Cys Thr Asn Trp
         100
Asn Ser Ser Ala Leu Ala Gln Lys Pro Tyr Ser Gly Arg Arg Pro Asp Ala Ile Arg
Leu Gly Leu Gly Asn His Asn Tyr Cys Arg Asn Pro Asp Arg Asp Ser Lys Pro Trp
                                                     150
Cys Tyr Val Phe Lys Ala Gly Lys Tyr Ser Ser Glu Phe Cys Ser Thr Pro Ala Cys
Ser Glu Gly Asn Ser Asp Cys Tyr Phe Gly Asn Gly Ser Ala Tyr Arg Gly Thr His
Ser Leu Thr Glu Ser Gly Ala Ser Cys Leu Pro Trp Asn Ser Met Ile Leu Ile Gly Lys
                   200
Val Tyr Thr Ala Gln Asn Pro Ser Ala Gln Ala Leu Gly Leu Gly Lys His Asn Tyr
Cys Arg Asn Pro Asp Gly Asp Ala Lys Pro Trp Cys His Met Leu Lys Asn Arg Arg
                                                               250
Leu Thr Trp Glu Tyr Cys Asp Val Pro Ser Cys Ser Thr Cys Gly Leu Arg Gln Tyr
Ser Gln Pro Gln Phe Arg Ile Lys Gly Gly Leu Phe Ala Asp Ile Ala Ser His Pro Trp
Gln Ala Ala Ile Phe Ala Lys His Arg Arg Ser Pro Gly Glu Arg Phe Leu Cys Gly
                                   300
Gly Ile Leu Ile Ser Ser Cys Trp Ile Leu Ser Ala Ala His Cys Phe Gln Glu Arg Phe
Pro Pro His His Leu Thr Val Ile Leu Gly Arg Thr Tyr Arg Val Val Pro Gly Glu Glu
Glu Gln Lys Phe Glu Val Glu Lys Tyr Ile Val His Lys Glu Phe Asp Asp Asp Thr
   350
Tyr Asp Asn Asp Ile Ala Leu Leu Gln Leu Lys Ser Asp Ser Ser Arg Cys Ala Gln
Glu Ser Ser Val Val Arg Thr Val Cys Leu Pro Pro Ala Asp Leu Gln Leu Pro Asp
                                           400
Trp Thr Glu Cys Glu Leu Ser Gly Tyr Gly Lys His Glu Ala Leu Ser Pro Phe Tyr
Ser Glu Arg Leu Lys Glu Ala His Val Arg Leu Tyr Pro Ser Ser Arg Cys Thr Ser
Gln His Leu Leu Asn Arg Thr Val Thr Asp Asn Met Leu Cys Ala Gly Asp Thr Arg
                   450
Ser Gly Gly Pro Gln Ala Asn Leu His Asp Ala Cys Gln Gly Asp Ser Gly Gly Pro
Leu Val Cys Leu Asn Asp Gly Arg Met Thr Leu Val Gly Ile Ile Ser Trp Gly Leu
                                                           500
Gly Cys Gly Gln Lys Asp Val Pro Gly Val Tyr Thr Lys Val Thr Asn Tyr Leu Asp
Trp Ile Arg Asp Asn Met Arg Pro
                   527
```

Fig. 2.

\* Site of cleavage in one-chain t-PA to give two-chain t-PA, in which the A-chain contains the two kringle regions and the B-chain contains the serine protease region.

*Fig.3.*

Prepro-Protein C

European Patent Office

EUROPEAN SEARCH REPORT

Application Number

EP 89 30 8330

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 191 606 (ELI LILLY) * Claims 1-31; page 35, lines 16-17; page 36, lines 1-13; page 38, lines 18-31 * | 1-12 | A 61 K 37/54 // (A 61 K 37/54 A 61 K 37:465) |
| Y | GB-A-2 176 703 (THE WELLCOME FOUNDATION) * Claims 1-5; figures 1,2 * | 1-12 | |
| Y | BIOLOGICAL ABSTRACTS/RRM 34018327, Biosis, Philadelphia, PA, US; J.C. FREDENBURCH et al.: "Enhancement by activated protein of TPA-induced lysis in a cell-free system", & THROMBOSIS AND HAEMOSTASIS(West Germany) 1987, vol. 58, no. 1, page 430 * Abstract * | 1-12 | |
| P,X | EP-A-0 318 201 (SCRIPPS CLINIC AND RESEARCH FOUNDATION) * Claims 1-5 * | 1,6,7, 10 | |
| P,X | WO-A-8 902 747 (THE AMERICAN NATIONAL RED CROSS) * Claim 1; page 4, lines 16-19; page 6, lines 18-23 * | 1,10 | TECHNICAL FIELDS SEARCHED (Int. Cl.5) A 61 K |

The present search report has been drawn up for all claims

| Place of search | Date of completion of the search | Examiner |
|---|---|---|
| THE HAGUE | 29-11-1989 | PEETERS J.C. |